# EUROPÄISCHE PATENTANMELDUNG

(11) **EP 0 780 418 A2**
(43) Veröffentlichungstag der Anmeldung: **25.06.1997**
(21) Anmeldenummer: 96119773.8
(22) Anmeldetag: 10.12.1996
(51) Int. Cl.: C08G 59/50, C08G 18/10, C08J 5/24, H01B 3/40, C07D 251/34

(54) **Triamine und ein Verfahren zu ihrer Herstellung**

(30) Priorität: 21.12.1995 DE 19548026
(71) Anmelder: BAYER AG, 51368 Leverkusen (DE)
(72) Erfinder: König, Klaus, Dr., 51519 Odenthal (DE); Neuner, Otto, Dr., 51465 Bergisch Gladbach (DE); Rasshofer, Werner, Dr., 51061 Köln (DE)

(57) **Zusammenfassung**

Beschrieben werden flüssige Polyaminvernetzer-Zubereitungen auf der Basis von trimerisiertem 2,4- und/oder 2,6-Toluylen-diisocyanat unter Ausbildung einer Isocyanuratstruktur und Hydrolyse der Isocyanatgruppen zu Aminogruppen. Die neuen Vernetzer-Zubereitungen sind gekennzeichnet durch einen Gehalt von 40 bis 80 % des Gesamtgewichts des Feststoffanteils der Vernetzer-Zubereitung an nicht weiter kondensiertem 1,3,5-Tris-(3-amino-4-methyl-phenyl,2-methyl-3-amino-phenyl)-isocyanurat (I) und durch einen Gehalt von höchstens 1,0 % des Gesamtgewichts der Vernetzer-Zubereitungen an Toluylen-2,4- bzw. -2,6-diamin (III). Zur Herstellung wird Toluylen-diisocyanat unter Ausbildung der Isocyanuratstruktur trimerisiert, bis 10 bis 25 % der NCO-Gruppen umgesetzt worden sind. Danach wird das nicht kondensierte Toluylen-diisocyanat abdestilliert, das dabei entstehende Sumpfprodukt unmittelbar in einem Lösungsmittel A aufgenommen und die dabei entstehende 30 bis 70 gew.-%ige Lösung der Hydrolyse in Wasser/N,N-Dialkyl-carbonsäureamid in Gegenwart einer katalytischen Menge eines basischen Katalysators eindosiert. Nach der Hydrolyse werden die Vernetzer-Zubereitungen durch anteiliges Abdestillieren von Lösungsmitteln und Zugabe eines Lösungsmittels B auf einen Feststoffanteil von 35 bis 60 % des Gesamtgewichts der Zubereitung eingestellt.

## Beschreibung

Die Erfindung betrifft eine flüssige Polyaminvernetzer-Zubereitung mit einem Feststoffanteil auf der Basis von trimerisiertem Toluylen-2,4- und/oder -2,6-diisocyanat, deren Isocyanatgruppen in Aminogruppen übergeführt wurden, mit einem hoben Gehalt des nicht weiter kondensierten Trimeren mit der Isocyanuratstruktur und einem sehr niedrigen Gehalt an Toluylen-2,4- bzw. -2,6-diamin. Die Erfindung betrifft weiterhin ein Verfahren zur Herstellung einer solchen Vernetzer-Zubereitung.

Polyaminvernetzer haben technische Bedeutung als Vernetzer und Härter zur Herstellung von Prepregs und von Harzmassen auf Epoxidbasis. Zur Herstellung von Prepregs werden Verstärkungsmaterialien mit Polyepoxidharzen und Lösungen der Vernetzer-Zubereitung imprägniert und können, gegebenenfalls nach einer Lagerung, unter Formgebung thermisch ausgehärtet werden. Bei der Herstellung von Harzmassen in den verschiedenen technischen Bereichen, insbesondere zur Einhüllung von Halbleiterbauelementen, elektronischen und elektrotechnischen Schaltungen, die ebenfalls thermisch gehärtet werden können, werden im allgemeinen Polyepoxidharze und Polyaminvernetzer in trockener Form miteinander vorvermischt, aufgeschmolzen und sodann als Gießharz eingesetzt. Man kann für diesen Zweck jedoch auch Lösungen von Polyepoxidharz und Vernetzer zur Anwendung bringen, wobei Lösungsmittel, wie Aceton, Ethylacetat, Methyl-ethylketon, 2-Methoxy-ethanol und andere, insbesondere die genannten Ketone, zum Einsatz gelangen. Lösungen (Zubereitungen) der Vernetzer für diese Anwendungsform enthalten typischerweise 40 bis 60 Gew.-% Vernetzer in der Gesamtlösung. Beim Vergießen solcher Lösungen wird das Lösungsmittel bei der Anwendungstemperatur, gegebenenfalls unter vermindertem Druck entfernt. Auch für diesen Anwendungsbereich sind die in Frage kommenden Polyepoxidharze und eventuelle weitere Stoffe, wie Füllstoffe, Farbstoffe und gegebenenfalls weitere Aminhärter-Komponenten dem Fachmann bekannt. Polyaminvernetzer, die den erfindungsgemäßen ähneln, und die in Frage kommenden Verwendungszwecke sind bereits bekannt, beispielsweise aus EP-A 271 772 und EP-A 274 646 zur Herstellung von Harzmassen und Prepregs aus Polyepoxidharzen.

Die Zielvorstellung zur Gewinnung eines Polyamins auf der Basis von Toluylendiisocyanat ist die der Kondensation zu einem Triisocyanat mit Isocyanuratstruktur und anschließende Überführung der NCO-Gruppen in Aminogruppen gemäß folgender Reaktionsgleichung:

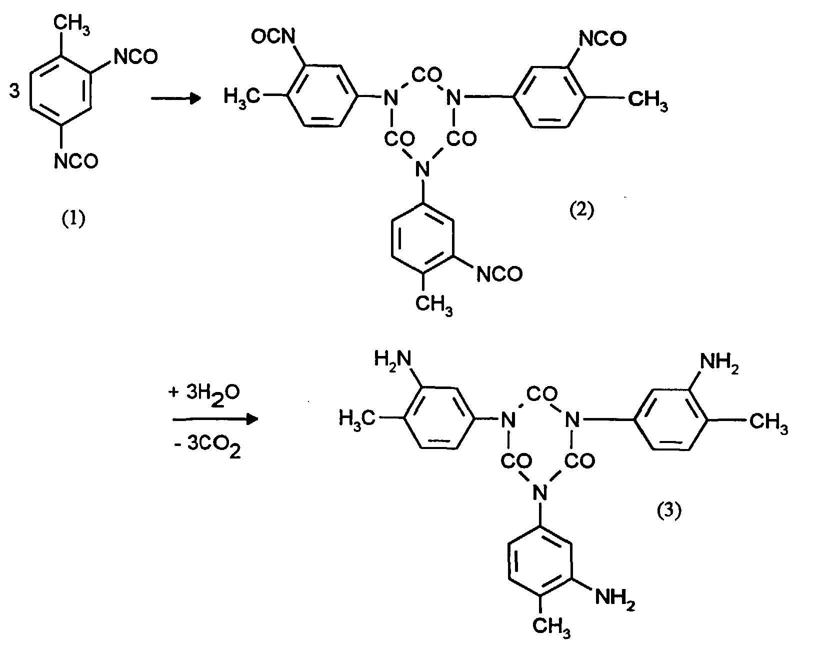

Hierbei wird am Beispiel des Toluylen-2,4-diisocyanats (1), Molekulargewicht 174,16, NCO-Gehalt 48,25 Gew.-%, die Trimerisierung zum 1,3,5-Tris-(3-Isocyanato-4-methyl-phenyl)-isocyanurat (2), Mol-Gewicht 522,49, NCO 24,13 Gew.-%, und dessen Hydrolyse unter Abspaltung von CO₂ zum korrespondierenden 1,3,5-Tris-(3-Amino-4-methyl-phenyl)-isocyanurat (3), Mol-Gewicht 444,50, NH₂-Gehalt 10,81 Gew.-%, gezeigt. (2) und seine Herstellung durch Trimerisierung von (1) ist aus US 2.801.244 bekannt. Die Verseifung von (2) zu (3) mit Wasser, beispielsweise in Dimethylformamid (DMF) als Lösungsmittel ist ebenfalls bereits bekannt, beispielsweise aus DE-A 32 27 219 und EP-A 271 772. Die oben skizzierte Reaktionsfolge von (1) über (2) zu (3) und ihre Darstellung in der oben genannten Patentliteratur sind jedoch lediglich idealisiert und werden in der Praxis nicht erreicht, da zwangsläufig zahlreiche polymerhomologe Reaktionen und Nebenreaktionen eintreten. So wird beobachtet, daß die Kondensation von beispielsweise (1) (analoges gilt auch für Stellungsisomere von (1), wie beispielsweise das Toluylen-2,6-diisocyanat) nicht beim Trimeren (2) der obigen idealisierten Darstellung haltmacht, sondern, daß die an den Substituenten des Isocyanuratringes vorhandenen NCO-Gruppen ihrerseits eine weitere Kondensation mit Diisocyanaten (1) oder gar mit weiteren bereits gebildeten Trimeren eingehen können. Dies ist, ebenfalls wieder vereinfacht und idealisiert, im folgenden dargestellt:

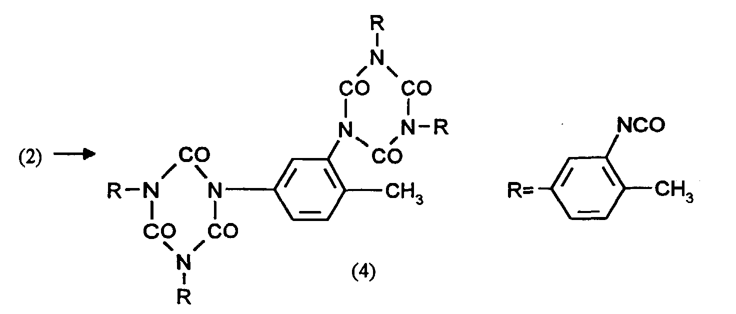

Eine solche weitergehende Kondensation findet selbstverständlich unter Verbrauch von Isocyanat-Gruppen statt, die dann nach der Hydrolyse auch als Amino-Gruppen nicht mehr vorhanden sein können. Hierdurch sinkt der NCO-Gehalt bzw. der nach der Hydrolyse gewünschte NH₂-Gehalt eines Polyaminvernetzers, wobei die Funktionalität der einzelnen höherkondensierten Moleküle steigt. In unerwünschter Weise wird hierbei aber auch durch die einhergehende Erhöhung des Molekulargewichtes solcher weiteren Kondensate die Viskosität des Vernetzers in einer unerwünschten und für den Einsatz schwierigen Form erhöht. Insgesamt sinkt also der Gehalt an Kondensaten mit nur einem Isocyanuratring, während gleichzeitig die Menge an weiteren Kondensaten mit 2 bis 6 Isocyanuratringen oder (wenn auch nur in untergeordnetem Maße) die Menge der weiteren Kondensate mit 7 bis 10 oder mehr Isocyanuratringen steigt (Kondensationsgrade 2 bis 6, 7 bis 10 oder höher). Neben der verschlechterten allgemeinen Handhabbarkeit infolge der durch die weitere Kondensation eintretenden Viskositätserhöhungen sinkt auch die Genauigkeit der analytischen Bestimmung der funktionellen Gruppen, die andererseits wegen der Unübersichtlichkeit der weiteren Kondensation die einzige Rezepturgrundlage bei den oben beschriebenen Anwendungen darstellt. Die Zugänglichkeit der funktionellen Gruppen, selbst wenn diese der analytischen Bestimmung noch zugänglich sind, sinkt aber im Vernetzer aus sterischen Gründen, so daß grundsätzlich vorhandene funktionelle Gruppen nicht in vollem Umfange an der Vernetzung/Aushärtung von Harzgemischen teilnehmen können.

Ein weiterer Nachteil von Vernetzern mit Anteilen hochfunktioneller (hochkondensierter) Moleküle tritt ein, wenn man in üblicher Weise ein Vorprodukt aus Epoxidharz und Vernetzer auf Glasfasermatten oder anderen Verstärkern herstellt und dies nach Abdampfen der Lösungsmittel durch Erhitzen zunächst nur in einen vorgehärteten Zustand (sogenannten "B-Zustand") bringt, um ein lagerfähiges Halbzeug zu gewinnen, das erst in einer 2. Stufe durch Verpressen in hocherhitzten Formen endgültig verarbeitet wird. Hierbei verursachen hochfunktionelle Vernetzeranteile einen zu engen Spielraum zwischen hoher Klebrigkeit, die das Entformen des Halbzeugs erschwert, und zu starker Vorvernetzung, die die Endverarbeitung stört.

Eine sehr empfindliche Stufe bei der Gewinnung von Polyaminvernetzern der hier beschriebenen Art ist weiterhin die Hydrolyse. Innerhalb kleiner Zeiteinheiten liegen nämlich neben noch nicht hydrolysiertem Isocyanat bereits durch Hydrolyse entstandene Aminogruppen vor. Diese Aminogruppen sind nun aber höchst reaktiv gegenüber Isocyanatgruppen, mit denen sie unter Ausbildung von Harnstoffgruppen reagieren. Dies wird in vereinfachter Form an folgendem Formelschema dargestellt:

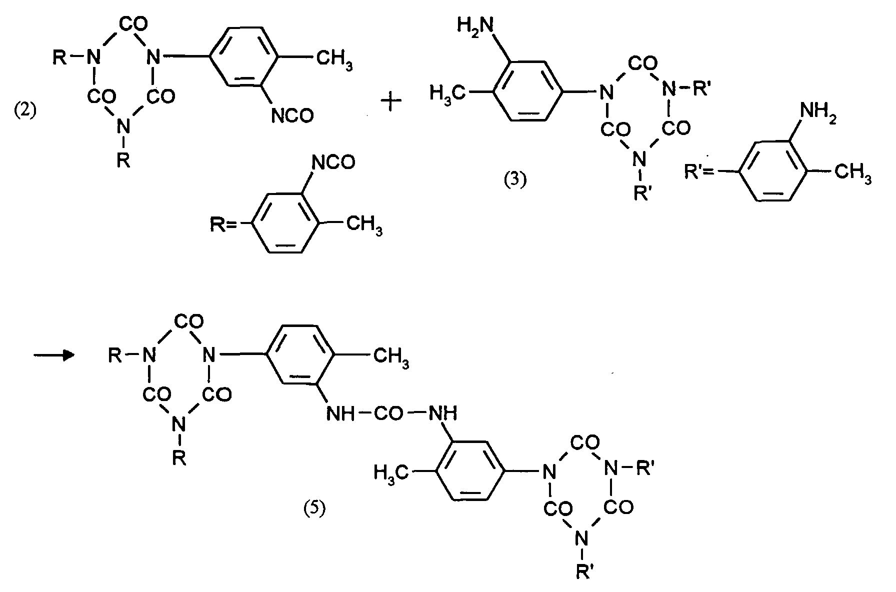

Auch diese Harnstoffbildung stellt eine empfindliche Einbuße an funktionellen Gruppen dar, wobei wiederum die Funktionalität der einzelnen Moleküle (hier (5) mit dem Kondensationsgrad 2) steigt, und ist daher ebenso geeignet, die Viskosität in unerwünschter Weise weiter nach oben zu treiben. Schließlich muß bei verschärften Hydrolysebedingungen auch mit der Möglichkeit der hydrolytischen Spaltung des Isocyanuratringes gerechnet werden, wodurch weitere Komplikationen eintreten.

Zur Vermeidung der beschriebenen unerwünschten Harnstoffbildung bei der Hydrolyse von (2) ist bereits beschrieben worden, Kondensate, die in idealisierter Form als Hauptkomponente (2) enthalten, mit Benzylalkohol zunächst zum zugehörigen Poly-benzylurethan umzusetzen, welches anschließend durch katalytische Hydrierung zum Polyamin der idealisierten Form (3), zu Toluol und CO₂ weiter umgesetzt wird (EP-A 0 048 369). Diese Möglichkeit ist jedoch sehr aufwendig und teuer und erfordert die Handhabung des systemfremden, hochsiedenden Stoffs Benzylalkohol.

Wollte man die oben beschriebene weitere Kondensation zu höhermolekularen und hochviskosen Kondensaten mit größerer Anzahl an Cyanuratringen zurückdrängen, müßte man die Kondensation früher unterbrechen. Dies birgt jedoch das Risiko des Gehalts an nicht in die Kondensation einbezogenem Toluylen-diisocyanat (2,4-bzw. 2,6-). Hieraus entsteht jedoch bei der Hydrolyse Toluylen-diamin, das, wie andere primäre aromatische Amine auch, gesundheitsgefährlich ist.

Die vorliegende Erfindung überwindet die genannten Nachteile durch Bereitstellung von Polyaminvernetzer-Zubereitungen mit einem erhöhten Gehalt an Trimeren (3), bezogen auf das Gesamtgewicht des Vernetzergemisches, bei gleichzeitig äußerst niedrigem Gehalt an Toluylen-diamin. Erst dadurch wird ihre technische Anwendung möglich, die bei bisher beschriebenen Polyaminvernetzern dieser Art noch nicht realisiert werden konnte.

Die Erfindung betrifft flüssige Polyaminvernetzer-Zubereitungen mit einem Feststoffanteil auf der Basis von Triaminen der Formel 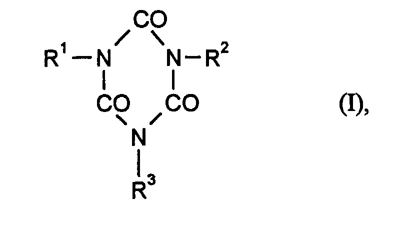 in der
- R¹, R² und R³: unabhängig voneinander 2-Methyl-3-amino-phenyl oder 3-Amino-4-methyl-phenyl bedeuten,
gebildet durch Hydrolyse des zugrundeliegenden trimerisierten Toluylen-diisocyanats der Formel 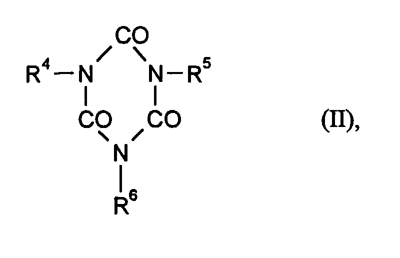 in der
- R⁴, R⁵ und R⁶: unabhängig voneinander 2-Methyl-3-isocyanato-phenyl oder 3-Isocyanato-4-methyl-phenyl bedeuten,
gekennzeichnet durch einen Gehalt von 40 bis 80 % des Gesamtgewichts des Feststoffanteils der Vernetzer-Zubereitungen an Triamin (I) mit dem durch die Zahl der Isocyanuratkerne ausgedrückten Kondensationsgrad 1 und durch einen Gehalt von höchstens 1,0 % des Gesamtgewichts des Feststoffanteils der Vernetzer-Zubereitungen an Toluylen-diaminen der Formel 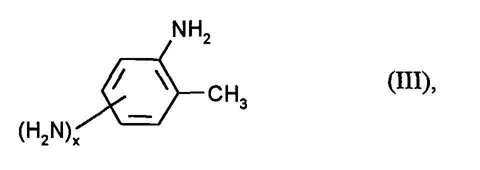 in der
- x: die 2- oder die 4-Position zur Methylgruppe anzeigt,
wobei der Rest zu 100 % des Feststoffanteils Kondensate mit höherem Kondensationsgrad darstellt, wobei der Feststoffanteil 35 bis 60 % des Gesamtgewichts der Zubereitungen beträgt und wobei der Rest zu 100 % des Gesamtgewichtes ein Lösungsmittelgemisch darstellt, das zu 20 bis 40 Gew.-% vom Lösungsmittelgemisch ein N,N-Dialkyl-carbonsäureamid und zum Rest ein Ester oder Keton ist.

In bevorzugter Weise haben die erfindungsgemäßen Vernetzer-Zubereitungen einen Gehalt von 50 bis 65 % des Gesamtgewichts des Feststoffanteils der Vernetzer-Zubereitungen an Triamin (I). In weiter bevorzugter Weise enthalten die erfindungsgemäßen Vernetzer-Zubereitungen einen Höchstgehalt von 0,8 %, besonders bevorzugt höchstens 0,5 %, an Diamin (III), bezogen auf das Gesamtgewicht des Feststoffanteils der Vernetzer-Zubereitungen. Die den Rest des Feststoffanteils darstellenden höheren Kondensate sind überwiegend solche mit Kondensationsgraden 2 und 3, rasch abfallende Mengen mit Kondensationsgraden 4 bis 6 und nur untergeordnete Mengen noch höherer Kondensate.

Dies steht in ausgeprägtem Gegensatz zu Vernetzern auf der Basis von (I) des Standes der Technik, die entweder einen Anteil an (I) von nur etwa 10 bis 20 Gew.-%, aber 80 bis 90 Gew.-% an höheren Kondensaten mit Kondensationsgraden von 2 bis über 10 haben oder die bei höheren Anteilen von (I) gleichzeitig einen zu hohen und arbeitshygienisch bedenklichen Anteil an (III) aufweisen.

Die erfindungsgemäßen Vernetzer-Zubereitungen liegen in Form einer klaren, 35 bis 60 gew.-%igen, bevorzugt 40 bis 55 gew.-%igen, Lösung vor, wobei das Lösungsmittel, bezogen auf die Gesamtmenge an Lösungsmittel, zu 20 bis 40 Gew.-%, bevorzugt 30 bis 35 Gew.-%, aus einem N,N-Dialkyl-carbonsäureamid und zu 60 bis 80 %, bevorzugt 65 bis 70 %, aus einem Keton oder einem Ester, bevorzugt solchen mit einem Siedepunkt von maximal 155°C, besteht.

Die Erfindung betrifft weiterhin ein Verfahren zur Herstellung von flüssigen Polyaminvernetzer-Zubereitungen der oben genannten Art, das dadurch gekennzeichnet ist, daß
a) in einem ersten Schritt Toluylen-diisocyanat der Formel 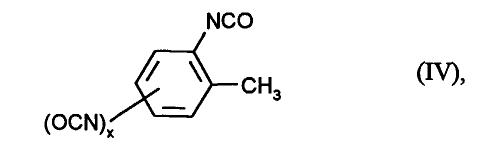 in der x die obige Bedeutung hat,
   bei einer Temperatur von 20 bis 100°C in Gegenwart eines ersten basischen Katalysators unter Ausbildung von Isocyanuratringen gemäß Formel (I) kondensiert wird, bis 10 bis 25 %, bevorzugt 12 bis 20 %, der NCO-Gruppen umgesetzt worden sind,
b) aus dem Kondensat von Schritt a) nach Desaktivieren des ersten basischen Katalysators mittels Lewis-Säuren das nichtkondensierte (IV) destillativ bei einer Heizmediumtemperatur von 150 bis 200°C und 0,1 bis 50 mbar bis zu einem Restgehalt von höchstens 1 %, bevorzugt höchstens 0,8 %, besonders bevorzugt höchstens 0,5 % der in Schritt a) eingesetzten Menge an (IV) abtrennt,
c) das Sumpfprodukt der Destillation von Schritt b) unmittelbar in einem Lösungsmittel A aus der Gruppe der Ester, der Ketone, der aromatischen Kohlenwasserstoffe oder Gemischen mehrerer von ihnen in einer solchen Menge aufnimmt, daß eine 30 bis 70 gew.-%ige Lösung des Sumpfproduktes entsteht,
d) die im Schritt c) erhaltene Lösung des Sumpfprodukts bei 70 bis 120°C, bevorzugt 80 bis 100°C, besonders bevorzugt 90 bis 95°C, in ein Gemisch aus Wasser, einer katalytischen Menge eines zweiten basischen Katalysators und einem N,N-Dialkyl-carbonsäureamid eindosiert und dabei NCO-Gruppen zu NH₂-Gruppen und CO₂ hydrolysiert, wobei die Menge an H₂O 100 bis 500 Mol-%, bevorzugt 200 bis 400 Mol-%, bezogen auf die Menge an NCO-Äquivalenten im Sumpfprodukt von Schritt b), beträgt und die Menge an N,N-Dialkyl-carbonsäureamid das 3- bis 15-fache, bevorzugt 5- bis 12-fache des Sumpfprodukts beträgt und
e) den Polyaminvernetzer durch anteiliges Abdestillieren der Lösungsmittel als konzentrierte Lösung gewinnt, die durch Zusatz eines Lösungsmittels B aus der Gruppe der Ketone und der Ester auf den oben genannten Feststoffgehalt eingestellt wird.

Als Toluylen-diisocyanat (IV) können erfindungsgemäß Toluylen-2,4- und -2,6-diisocyanat und ihre Gemische, insbesondere in ihren technisch verfügbaren Qualitäten eingesetzt werden.

Die Kondensation im Verfahrensschritt a) wird bei einer Temperatur von 20 bis 100°C, bevorzugt von 40 bis 60°C, in Gegenwart eines ersten basischen Katalysators aus der Gruppe von Alkalimetallhydroxiden, -carbonaten und -C₁-C₁₀-carboxylaten, sowie von quartären Ammoniumbasen, Phosphoniumbasen und von Mannich-Basen durchgeführt. Beispiele für solche erste basische Katalysatoren sind: NaOH, KOH, Na₂CO₃, K₂CO₃, HCOONa, HCOOK, CH₃COONa, CH₃COOK und Na- und K-Salze weiterer aliphatischer Carbonsäuren mit bis zu 10 C-Atomen, ferner Tetramethyl-, Tetraethyl-, Tetrabutylammoniumhydroxid, quartäre Ammoniumhydroxide mit einem langen C₆-C₁₈-Alkylrest, dem Phenyl- oder Benzylrest und 3 C₁-C₄-Alkylresten, die auch verschieden untereinander sein können, ferner quartäre Ammoniumhydroxide mit 2 langkettigen C₆-C₁₈-Alkylresten, 2 Phenyl- oder Benzylresten und 2 C₁-C₄-Alkylresten, ferner quartäre Ammoniumhydroxide, die Hydroxyalkylgruppen oder Ethergruppen mit endständigem Hydroxyl enthalten, ferner die aus Phenol, substituiertem Phenol, Bisphenolen und Ketonen durch Umsetzung mit Formaldehyd und Ammoniak, primären oder sekundären Aminen erhältlichen Mannich-Basen, wie HO-C₆H₄-CH₂-N(CH₃)₂ (Houben-Weyl, Methoden der Organischen Chemie, Band XI/1 (1957), S. 756 ff; DE-A 25 51 634). Alle diese basischen Katalysatoren sind dem Fachmann bekannt. In bevorzugter Weise werden die Mannich-Basen und die quartären Ammoniumhydroxide, besonders bevorzugt die Mannich-Basen eingesetzt. Katalytische Mengen sind hierbei 0,0005 bis 0,01 Äquivalent basischer Katalysator, bevorzugt 0,001 bis 0,005 Äquivalente, pro NCO-Äquivalent.

Die Kondensation von (IV) im Verfahrensschritt a) wird soweit fortgesetzt, bis 10 bis 25 %, bevorzugt 12 bis 20 % der NCO-Gruppen umgesetzt worden sind; dies kann durch Probenahme aus dem Reaktionsgemisch und Analyse sichergestellt werden.

Danach wird in einem Verfahrensschritt b) der erste basische Katalysator mittels Lewis-Säuren oder Alkylierung-/Acylierungsmitteln, wie o-/p-Toluolsulfonsäuremethylester, Dimethylsulfat, Benzoylchlorid, Acetylchlorid oder analogen Verbindungen, in einer äquivalenten oder einer bis zu 20 Äquivalent-% darüber liegenden Menge deaktiviert, und das im Schritt a) nicht kondensierte (IV) wird bis zu einem Restgehalt von höchstens 1 %, bevorzugt höchstens 0,8 %, besonders bevorzugt höchstens 0,5 % seiner Anfangsmenge abdestilliert. Hierzu wird bei einer Temperatur für das Heizmedium in der Destillationsapparatur mit Heizmantel, Heizschlangen oder ähnlicher indirekter Heizung von 150 bis 200°C, bevorzugt 160 bis 180°C, und bei einem Druck von 0,1 bis 50 mbar, bevorzugt 0,1 bis 20 mbar gearbeitet. Die Destillation kann einstufig oder mehrstufig bei fallenden Drücken in den verschiedenen Stufen durchgeführt werden. Für diese destillative Abtrennung kann beispielsweise ein Dünnschichtverdampfer, ein Spiralverdampfer oder eine ähnliche kontinuierlich betriebene Destillationsapparatur eingesetzt werden.

Das im Reaktionsschritt b) anfallende Sumpfprodukt wird im Reaktionsschritt c) unmittelbar in einem Lösungsmittel A aufgenommen. Das Lösungsmittel A ist eines aus der Gruppe aliphatischen Ester, der aliphatischen Ketone, der aromatischen Kohlenwasserstoffe oder einem Gemisch mehrerer von ihnen, bevorzugt mit einem Siedepunkt von höchstens 155°C. Beispiele sind Ethylacetat, Butylacetat, Methylpropionat, Aceton, Methylethylketon (MEK), Methylpropylketon, Methylbutylketon, Benzol, Toluol und die isomeren Xylole. Bevorzugte Lösungsmittel A sind Ester und Ketone, besonders bevorzugt Ethylacetat und MEK. Die Menge des Lösungsmittels A wird so bemessen, daß eine 30 bis 70 gew.-%ige Lösung des Sumpfproduktes aus Stufe b) entsteht.

Die im Reaktionsschritt c) erhaltene Lösung des Sumpfproduktes wird nunmehr im Reaktionsschritt d) bei 70 bis 120°C, bevorzugt 80 bis 100°C, besonders bevorzugt 90 bis 95°C ,in ein Gemisch aus Wasser, einer katalytischen Menge eines zweiten basischen Katalysators und eines N,N-Dialkyl-carbonsäureamids, wie Dimethylformamid (DMF), Diethylformamid, Dimethylacetamid (DMAc), bevorzugt DMF, eingetragen. Der zweite basische Katalysator ist ein Alkalimetallhydroxid, -carbonat oder C₁-C₁₀-carboxylat der oben genannten Art oder ein Alkalimetallhydrogencarbonat. Hierbei werden die NCO-Gruppen zu NH₂-Gruppen und CO₂ hydrolisiert. Die Wassermenge im Reaktionsschritt d) beträgt 100 bis 500 Mol-%, bevorzugt 200 bis 400 Mol-%, bezogen auf die Menge an NCO-Äquivalenten im Sumpfprodukt von Schritt b). Die katalytische Menge an zweitem basischen Katalysator beträgt beispielsweise 0,0005 bis 0,01 Äquivalent, bevorzugt 0,001 bis 0,005 Äquivalent, an zweitem basischen Katalysator pro NCO-Äquivalent im Sumpfprodukt von b). Die Menge des N,N-Dialkyl-carbonsäureamids beträgt das 3- bis 15-fache, bevorzugt das 5- bis 12-fache des Sumpfprodukts.

Zur Durchführung der Hydrolyse im Reaktionsschritt d) wird das Gemisch aus Wasser, dem zweiten basischen Katalysator und dem N,N-Dialkyl-carbonsäureamid vorgelegt und auf die gewünschte Reaktionstemperatur gebracht. Unter gutem Rühren wird sodann die Lösung aus Sumpfprodukt vom Reaktionsschritt b) im Lösungsmittel A so eindosiert, daß die sofort einsetzende CO₂-Entwicklung gut beherrscht wird. Nach Beendigung der CO₂-Entwicklung und einer Nachreaktionszeit erhält man eine klare Lösung.

Eine zur Herstellung von Prepregs oder Harzmassen geeignete erfindungsgemäße Polyaminvernetzer-Zubereitung erhält man sodann im Schritt e) aus der anfallenden Hydrolyselösung durch anteiliges Abdestillieren der Lösungsmittel A und des N,N-Dialkyl-carbonsäureamids, wobei etwa überschüssiges Hydrolysewasser ebenfalls abdestilliert. Hierbei läßt man eine etwa 70 bis 85 gew.-%ige Lösung des Hydrolyseproduktes entstehen, die durch Zugabe eines Lösungsmittels B aus der Gruppe der Ketone und Ester der oben genannten Art auf den obengenannten Feststoffgehalt gebracht wird. Als solche gut handhabbare Zubereitungen sind für den praktischen Einsatz besonders die bevorzugt, bei denen DMF und MEK (als Lösungsmittel B) vorliegen.

Die erfindungsgemäßen Polyaminvernetzer-Zubereitungen weisen einen hohen Anteil von mindestens 40 % des Gesamtgewichts des Feststoffanteils an Triamin (I) mit nur einem Isocyanuratring auf. Gleichzeitig enthalten sie höchstens 1 % des Gesamtgewichts des Feststoffanteils an Toluylen-diamin (III). Der hohe Anteil an Einkern-Isocyanurat-Komponenten bewirkt eine niedrige Viskosität, die einerseits eine höhere Menge an funktionellen Gruppen mit sich bringt, verglichen mit Produkten, die nach Verfahren des Standes der Technik hergestellt worden sind. Diese höhere Anzahl an funktionellen Gruppen erlaubt einen sparsamen Einsatz der Polyaminvernetzer-Zubereitungen und damit kostengünstigere Rezepturen von Epoxidharzgemischen. Die geringere Viskosität verhindert weiterhin eine vorzeitige Verfestigung bei noch geringem Umsetzungsgrad zwischen Epoxid- und Amingruppen in den genannten Harzgemischen. Die erfindungsgemäße Herstellung der Polyaminvernetzer-Zubereitungen erlaubt einen hohen Wiedergewinnungsgrad der nicht in den flüssigen Zubereitungen verbleibenden Lösungsmittel.

### Beispiele

### Beispiel 1a (Kondensation)

1000 Gew.-Teile eines Gemisches aus 65 Gew.-% 2,4- und 35 Gew.-% 2,6-Toluylendiisocyanat und 0,26 Gew.-Teile der aus Phenol, Dimethylamin und Formaldehyd hergestellten Mannich-Base wurden bei 45°C gemischt. Die sofort einsetzende Trimerisation wurde bei 45 ± 2°C unter Rühren und Feuchtigkeitsausschluß solange fortgeführt, bis der NCO-Gehalt von ursprünglich 48,25 auf 42 Gew.-% abgesunken war. Durch Zugabe von 0,65 Gew.-Teile o-/p-Toluolsulfonsäuremethylester und 1-stündigem Nachrühren bei 60°C wurde die Trimerisation gestoppt. Mittels eines Dünnschichtverdampfers wurde das überschüssige monomere Isocyanat im Vakuum abdestilliert. Der erhaltene Destillationssumpf enthielt 0,3 Gew.-% freies monomeres Toluylendiisocyanat und 72 Gew.-% Tris-(isocyanatotoluol-)isocyanurat vom Kondensationsgrad 1. Der Destillationssumpf wurde unmittelbar in der gleichen Menge Ethylacetat aufgenommen.

### Beispiel 1b (Hydrolyse)

In einem 10-l-Reaktionsgefäß mit Rührer, Thermometer und einem wirksamen Rückflußkühler wurden 7300 g Dimethylformamid (DMF), 270 g vollentsalztes Wasser (15 mol) und 0,8 g NaOH in Form einer 50 %igen wäßrigen Lösung vorgelegt und auf 95 bis 100°C unter kräftigem Rühren erhitzt. Innerhalb von etwa 1 Stunde wurden hierzu 897,5 g Kondensat (im wesentlichen Trimerisat des Kondensationsgrades 1; 5 Äquivalente NCO) in Form einer 50 %igen Lösung in Ethylacetat zudosiert. Nach Beendigung der CO₂-Entwicklung wurde noch 15 Minuten nachgerührt und dann auf 70°C abgekühlt. Durch Destillation bei 200 mbar wurde sodann das Ethylacetat in Form eines Azeotrops mit der Zusammensetzung 91,9 % Ethylacetat und 8,1 % Wasser entfernt. Das Destillat trennte sich in zwei Phasen, von denen die obere Phase 96,6 % Ethylacetat und 3,4 % Wasser enthielt und durch Andestillieren vom Wasser befreit werden konnte, so daß das erhaltene Ethylacetat erneut in das Verfahren eingebracht werden konnte. Die untere Phase enthielt neben 90,4 % Wasser noch 8,4 % Ethylacetat, 1 % DMF und weitere nicht identifizierte Stoffe. Auch diese Phase wurde nach mehreren Versuchsläufen gesammelt und auf organische Anteile aufgearbeitet. Somit betrug die Ausbeute an Ethylacetat in einer Reihe von Folgeversuchen nahezu 100 %. Der Destillationssumpf wurde durch weitere Vakuumdestillation von der größten Menge an DMF befreit (das DMF konnte ohne weitere Reinigung in einem Folgeansatz verwendet werden), so daß eine Lösung des Polyaminvernetzers in DMF von 75 % Feststoffgehalt erhalten wurde. Als Destillationsbedingungen wurden hierbei zuletzt 90 bis 100°C/10 mbar erreicht. Das Vakuum wurde durch Zugabe von Stickstoff in den Destillationskolben aufgehoben. Nach Zugabe von 520 g Methylethylketon erhielt man eine Lösung des Polyamin-Vernetzergemisches von etwa 50 Gew.-% Feststoff, 16,6 Gew.-% DMF und 33,4 % MEK. Die Lösung hatte eine Aminzahl von 158, was für das Polyamin-Vernetzergemisch eine Aminzahl von 316 oder einen Gehalt von 9,0 % NH₂ bedeutet. Die Viskosität der Lösung betrug bei 25°C 150 mPas. Die Lösung hatte eine schwach bräunliche Farbe.

## Patentansprüche

1. Flüssige Polyaminvernetzer-Zubereitungen mit einem Feststoffanteil auf der Basis von Triaminen der Formel (I) 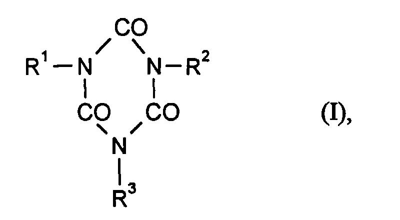 in der
R¹, R² und R³ unabhängig voneinander 2-Methyl-3-amino-phenyl oder 3-Amino-4-methyl-phenyl bedeuten,
gebildet durch Hydrolyse des zugrundeliegenden trimerisierten Toluylendiisocyanats der Formel 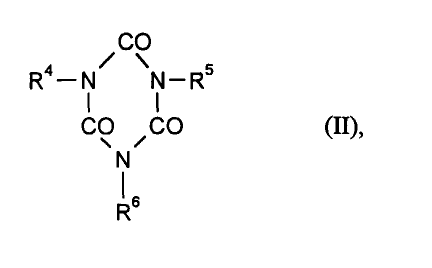 in der
R⁴, R⁵ und R⁶ unabhängig voneinander 2-Methyl-3-isocyanato-phenyl oder 3-Isocyanato-4-methyl-phenyl bedeuten,
gekennzeichnet durch einen Gehalt von 40 bis 80 % des Gesamtgewichts des Feststoffanteils der Vernetzer-Zubereitungen an Triamin (I) mit dem durch die Zahl der Isocyanuratkerne ausgedrückten Kondensationsgrad 1 und durch einen Gehalt von höchstens 1,0 % des Gesamtgewichts des Feststoffanteils der Vernetzer-Zubereitungen an Toluylen-diaminen der Formel 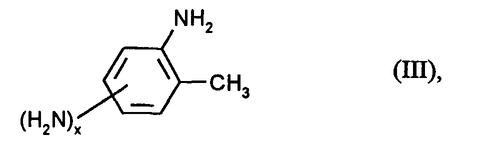 in der
x die 2- oder die 4-Position zur Methylgruppe anzeigt,
wobei der Rest zu 100 % des Feststoffanteils Kondensate mit höherem Kondensationsgrad darstellt, wobei der Feststoffanteil 35 bis 60 % des Gesamtgewichts der Zubereitungen beträgt und wobei der Rest zu 100 % des Gesamtgewichts ein Lösungsmittelgemisch darstellt, das zu 20 bis 40 Gew.-% vom Lösungsmittelgemisch ein N,N-Dialkyl-carbonsäureamid und zum Rest ein Ester oder Keton ist.

2. Vernetzer-Zubereitungen nach Anspruch 1, gekennzeichnet durch einen Gehalt von 50 bis 65 Gew.-% des Feststoffanteils an Triamin (I).

3. Vernetzer-Zubereitungen nach Anspruch 1, gekennzeichnet durch einen Gehalt von höchstens 0,8 %, bevorzugt von höchstens 0,5 %, des Gesamtgewichts des Feststoffanteils an Toluylen-diamin der Formel (III).

4. Vernetzer-Zubereitungen nach Anspruch 1, dadurch gekennzeichnet, daß der Feststoffanteil 40 bis 55 % des Gesamtgewichts der Zubereitungen beträgt.

5. Vernetzer-Zubereitungen nach Anspruch 1, dadurch gekennzeichnet, daß das Lösungsmittelgemisch zu 30 bis 35 Gew.-% ein N,N-Dialkyl-carbonsäureamid, bevorzugt N,N-Dimethylformamid, ist und zum Rest ein Ester oder Keton, bevorzugt Ethylacetat und Methyl-ethylketon ist.

6. Verfahren zur Herstellung von flüssigen Polyaminvernetzer-Zubereitungen gemäß Anspruch 1, dadurch gekennzeichnet, daß
a) in einem ersten Schritt Toluylen-diisocyanat der Formel 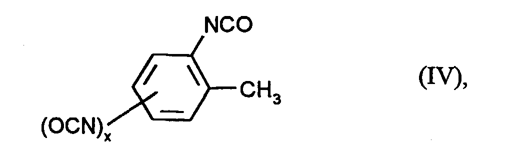 in der x die in Anspruch 1 genannte Bedeutung hat,
bei einer Temperatur von 20 bis 100°C in Gegenwart eines ersten basischen Katalysators unter Ausbildung von Isocyanuratringen gemäß Formel (I) von Anspruch 1 kondensiert wird, bis 10 bis 25 %, bevorzugt 12 bis 20 % der NCO-Gruppen umgesetzt worden sind,
b) aus dem Kondensat von Schritt a) nach Desaktivieren des ersten basischen Katalysators mittels Lewis-Säuren das nichtkondensierte (IV) destillativ bei einer Heizmediumtemperatur von 150 bis 200°C und 0,1 bis 50 mbar bis zu einem Restgehalt von höchstens 1 %, bevorzugt höchstens 0,8 %, besonders bevorzugt höchstens 0,5 % der in Schritt a) eingesetzten Menge an (IV) abtrennt,
c) das Sumpfprodukt der Destillation von Schritt b) unmittelbar in einem Lösungsmittel A aus der Gruppe der Ester, der Ketone, der aromatischen Kohlenwasserstoffe oder Gemischen mehrerer von ihnen in einer solchen Menge aufnimmt, daß eine 30 bis 70 gew.-%ige Lösung des Sumpfproduktes entsteht,
d) die im Schritt c) erhaltene Lösung des Sumpfprodukts bei 70 bis 120°C, bevorzugt 80 bis 100°C, besonders bevorzugt 90 bis 95°C, in ein Gemisch aus Wasser, einer katalytischen Menge eines zweiten basischen Katalysators und einem N,N-Dialkyl-carbonsäureamid eindosiert und dabei NCO-Gruppen zu NH₂-Gruppen und CO₂ hydrolysiert, wobei die Menge an H₂O 100 bis 500 Mol-%, bevorzugt 200 bis 400 Mol-%, bezogen auf die Menge an NCO-Äquivalenten im Sumpfprodukt von Schritt b), beträgt und die Menge an N,N-Dialkyl-carbonsäureamid das 3- bis 15-fache, bevorzugt das 5- bis 12-fache des Sumpfprodukts beträgt und
e) den Polyaminvernetzer durch anteiliges Abdestillieren der Lösungsmittel als konzentrierte Lösung gewinnt, die durch Zusatz eines Lösungsmittels B aus der Gruppe der Ketone und der Ester auf den in Anspruch 1 genannten Feststoffgehalt eingestellt wird.

7. Verfahren nach Anspruch 6, dadurch gekennzeichnet, daß die Destillation im Schritt b) bei einer Heizmediumtemperatur von 160 bis 180°C durchgeführt wird.

8. Verfahren nach Anspruch 6, dadurch gekennzeichnet, daß die Destillation im Schritt b) bei 0,1 bis 20 mbar durchgeführt wird.

9. Verfahren nach Anspruch 6, dadurch gekennzeichnet, daß das anteilige Abdestillieren der Lösungsmittel im Schritt e) eine 70 bis 85 gew.-%ige Lösung des Hydrolyseproduktes ergibt.
